# EUROPEAN PATENT APPLICATION

(11) **EP 1 529 547 A1**
(43) Date of publication of application: **11.05.2005**
(21) Application number: 04256880.8
(22) Date of filing: 05.11.2004
(51) Int. Cl.: A61M 16/00, A61H 31/00

(54) **Automatic ventilator for cardio pulmonary resucitation with chest compression timer and ventilation alarms**

(30) Priority: 05.11.2003 US 700515
(71) Applicant: O*Two Medical Technologies Inc., Mississauga, Ontario L5S 1C8 (CA)
(72) Inventor: Bowden, Kevin DJ, c/o O-Two Systems Int. Inc., Mississauga, Ontario L5S 1C8 (CA); Laswick, Ronald A, c/o O-Two Systems Int. Inc., Mississauga, Ontario L5S 1C8 (CA)
(74) Representative: McKechnie, Neil Henry

(57) **Abstract**

An automatic ventilator (10) for cardio-pulmonary resuscitation (CPR) comprising: an automatic ventilating circuit adapted for delivering two cycles of positive pressure breathable gas flow ventilation to a patient's airway; and a CPR timing circuit adapted to emit timed signals over a CPR period, after said two cycles, to guide an operator to time chest compressions applied to a patient.

## Description

### TECHNICAL FIELD

The invention relates to an automatic ventilator designed for use by minimally trained rescuers for the provision of Cardio Pulmonary Resuscitation (CPR) to patients in Cardiac and/or Respiratory Arrest.

### BACKGROUND OF THE ART

An analysis of published scientific works reviewing patient outcomes following cardiac arrest has shown that the average survival rate is no greater than 5%. This rate has not changed appreciably in recent years.

Published scientific data suggests that basic life support, if provided by bystanders, widens the opportunity for successful patient outcomes. Due to the reluctance of lay rescuers to perform expired air resuscitation, because of fear of infection or for aesthetic reasons, and the unsatisfactory performance of CPR due to the complexity of the techniques taught, and the poor retention of said techniques, does not provide for the best possible patient prognosis in all cases. Scientific evidence has also shown that longer periods of chest compressions, interposed with ventilations, improves cardiac output.

The invention provides an automatically cycled ventilator to provide both timing for chest compressions, to assist the rescuer in their techniques, and the required ventilations interposed between the sets of chest compressions. The current guidelines for such treatments are two ventilations followed by 15 chest compressions. The periodicity of the ventilations and chest compressions are currently established at 2 ventilations at an inspiratory to expiratory ratio I:E of 1:2 with a 2 second inspiratory time and 4 second expiratory time and 15 compressions provided at a rate of 100 compressions per minute, provided in 9 seconds. These ratios, periodicities and rates may change over time as scientific evidence is improved however, the device that is the subject of this application is so designed as to allow changes in calibration to meet any change in these requirements.

The invention may provide for monitoring the ventilations and warning the operator of any problem with the airway or deficiency in the provided volume due to mask leakage or airway obstruction with both visual and audible warnings.

A visual and audible timing mechanism may be included so that the rescuer can provide chest compressions at the required compression rate of 100 compressions per minute in sets of 15 compressions.

### DISCLOSURE OF THE INVENTION

The invention relates to a device designed to be powered pneumatically, by the regulated pressure from a medical oxygen gas cylinder where all functions including the visual and audible alarms are pneumatically powered. It is understood that other embodiments of this device may also be designed to operate electronically, using an electrical supply such as battery or mains and microprocessor to control the ventilator function as well as the alarm mechanisms. In addition, the current alarm and timing audible signals may be replaced with computer generated voice prompts.

Further details of the invention and its advantages will be apparent from the detailed description and drawings included below.

### BRIEF DESCRIPTION OF THE DRAWINGS

In order that the invention may be readily understood, one preferred embodiment of the invention will be described by way of example, with reference to the accompanying drawings wherein:

Figure 1 is a perspective drawing of the external layout of the device with all hoses and patient connections attached.

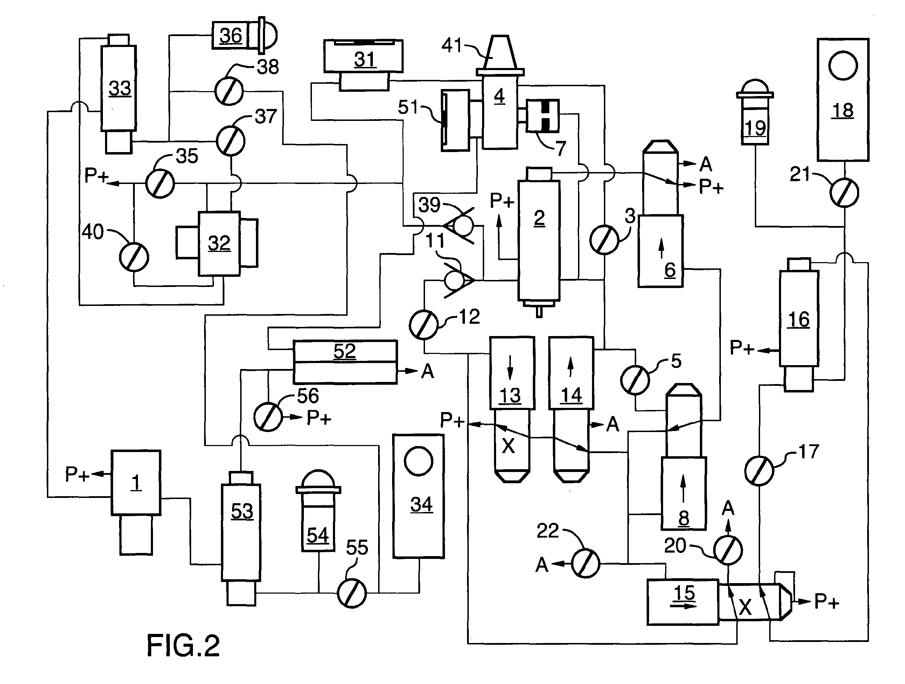

Figure 2 is a circuit diagram of the control and alarm circuits within the device

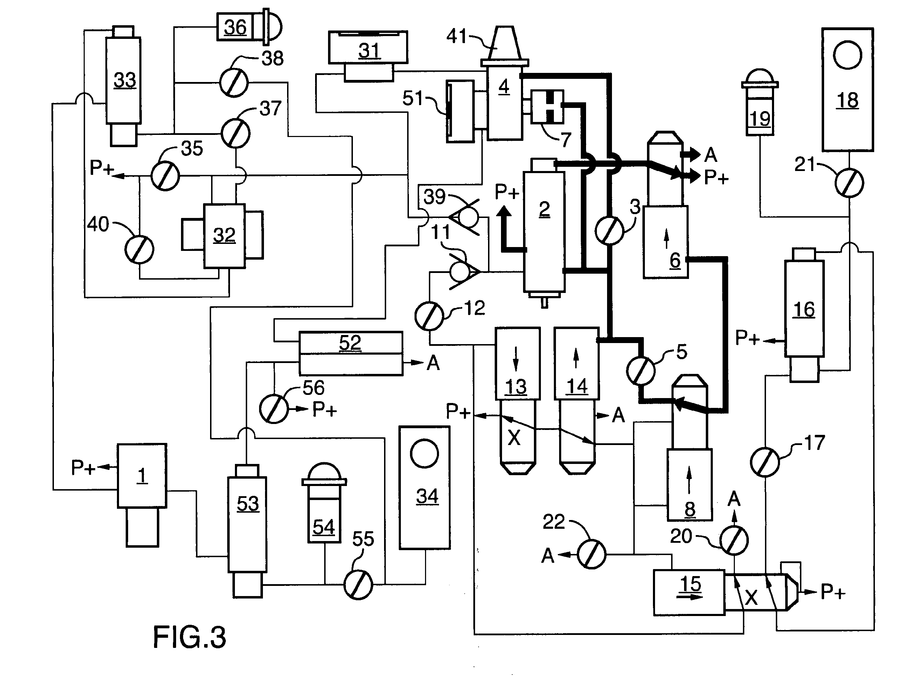

Figure 3 is a block diagram of the device detailing gas flow paths during the function of the device in the ventilation mode

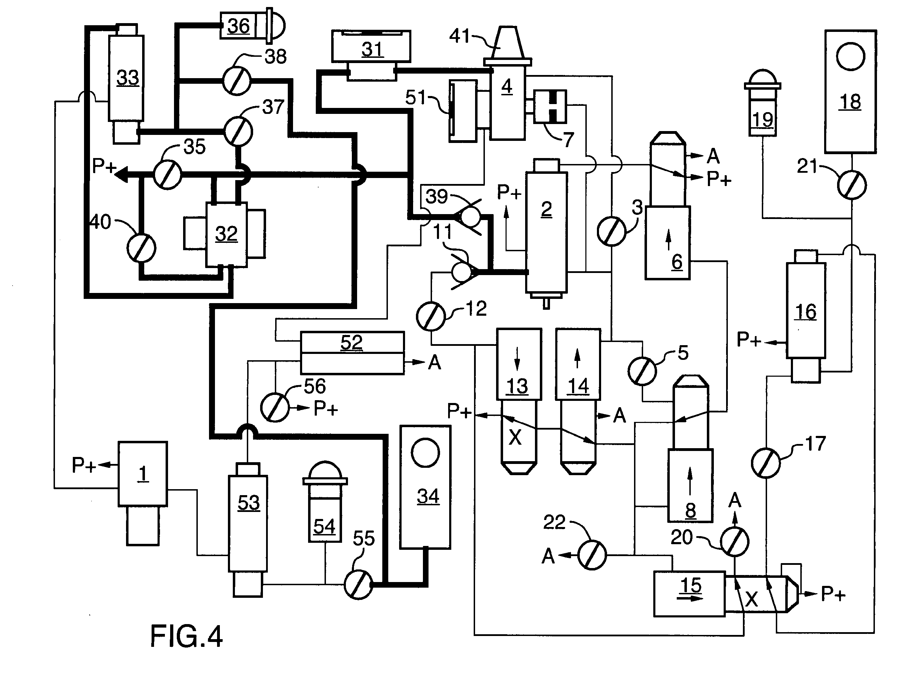

Figure 4 is a block diagram of the device showing the gas flow paths during the actuation of the mask leakage alarm

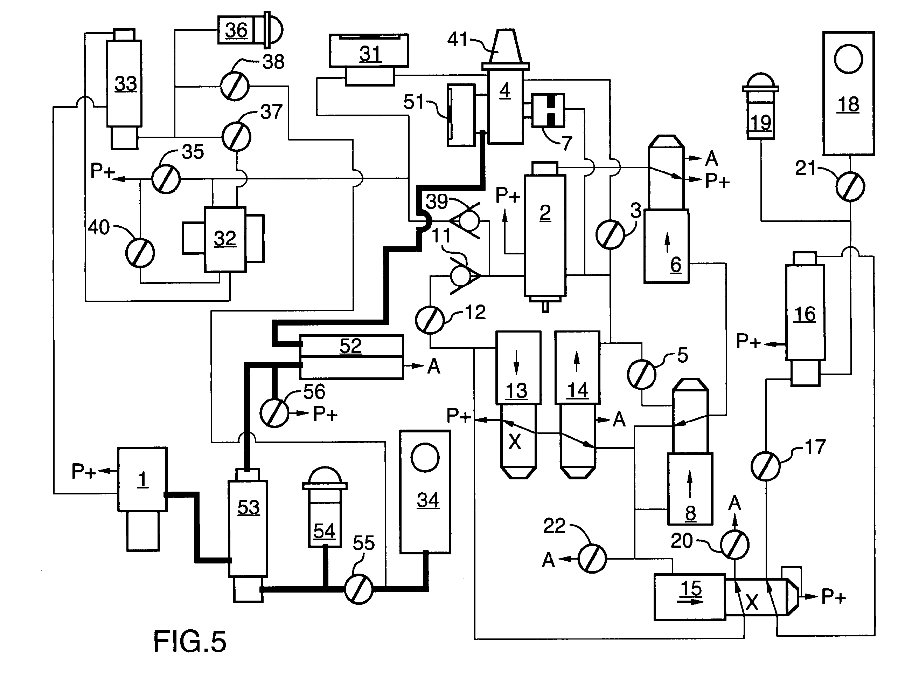

Figure 5 is a block diagram of the device showing the gas flow paths during the actuation of the airway obstruction alarm and

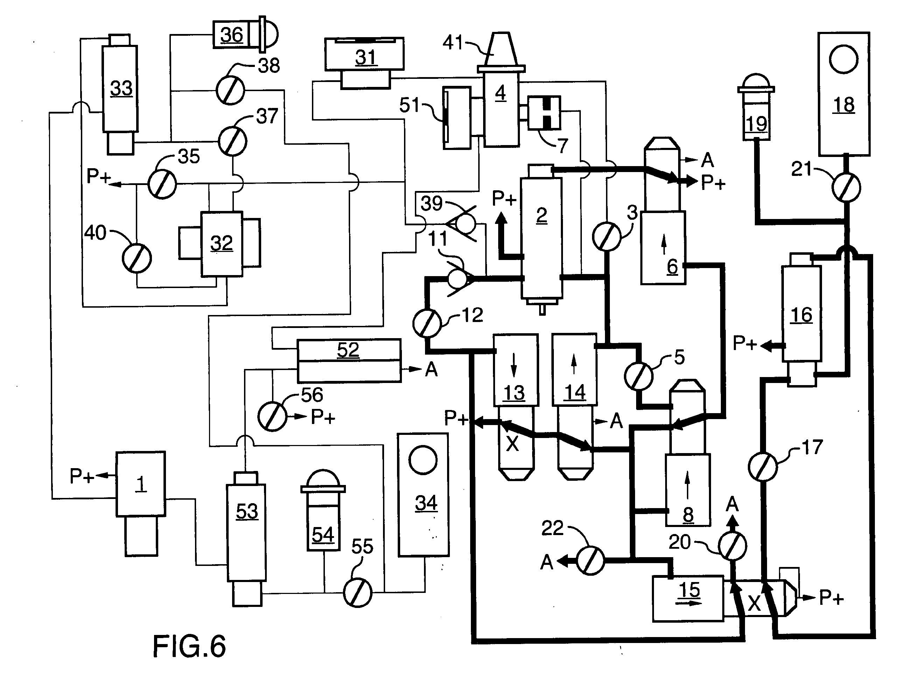

Figure 6 is a block diagram of the device showing the gas flow paths during the chest compression phase of the device and the actuation of the visual and audible timing mechanism.

Further details of the invention will become apparent from the detailed description presented below.

### DETAILED DESCRIPTION OF PREFERRED EMBODIMENTS

The device provides for a two phase operation of automatic ventilation followed by a timing device for chest compressions and alarms to detect and warn the operator of facemask leakage or airway obstruction.

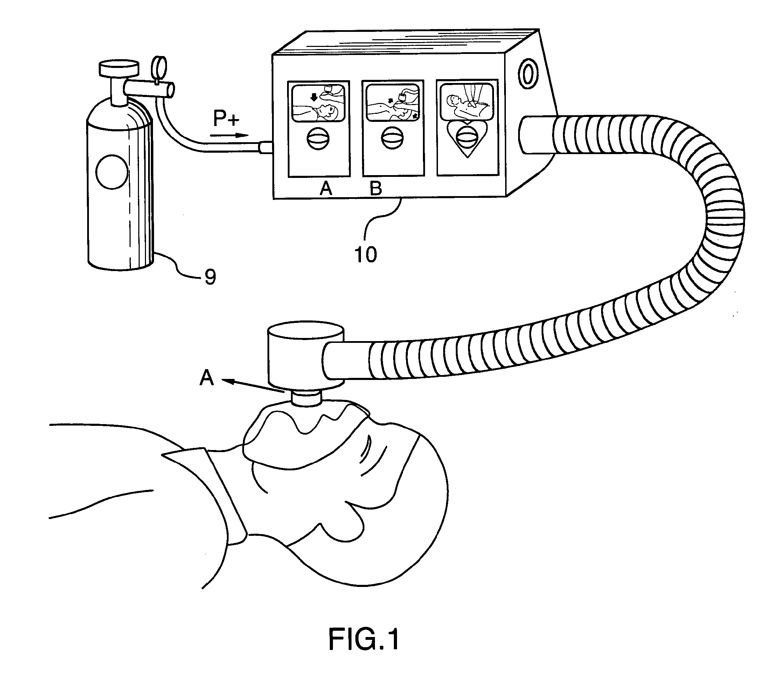

With reference to Figure 1, the breathable gas for ventilation is provided in a conventional cylinder 9 delivered to the automatic ventilator 10 and conveyed via a hose and face mask to the patient. In the embodiment described herein, the ventilator 10 is powered solely by the pressure of the gas in the cylinder 9 as gas passes through the ventilator to the patient and to ambient atmosphere. However it will be understood that the ventilator can be of the electronic type as well.

In the schematic drawings, the thicker lines indicate a relatively high rate of gas flow, whereas the thinner lines indicate a relatively low bleed gas flow, or no flow at all. The symbol "P+" is used to indicate connection to the pressure source (cylinder 9) and "A" indicates connection or venting to atmosphere. Alarms and signals emitted by the ventilator may be visual signals, audible signals, or vibratory signals and include electrically generated audible voice instructions.

### Automatic Ventilation Phase

The Automatic Ventilator Phase may be configured to provide two (2) cycles of controlled positive pressure oxygen flow ventilation with an Inspiratory time period of 2 seconds and expiratory time period of 4 seconds (I:E Ratio of 1:2) and a delivered tidal volume of 0.5L per cycle. A conventional automatic ventilator is described in the inventor's U. S. Patent 6,055,981.

This circuit consists of Internal Regulator (1), Main Switch (2), Automatic Flow Needle Valve (3), Patient Output Connector (4), Automatic Frequency Needle Valve (5) , Timing/Pressure Switch (6), Emergency Air Intake/Anti-Lockup Valve (7) , and 9 Second Delay Switch (8).

The Internal Regulator (1) reduces and stabilizes the pressure from a source cylinder pressure regulator (typically set to between 50-60 PSI) down to approximately 44 PSI (no flow). Gas from the Internal Regulator (1) flows through the Main Switch (2) (which is spring biased normally open) and then to the Automatic Flow Needle Valve (3) which reduces the flow rate to a fixed value, and then to the patient via the Patient Output Connector (4). The ventilator is now in the Inspiratory or "ON" state. The Automatic Flow Needle Valve (3) generates a back pressure which directs a portion of the pressure to the Automatic Frequency Needle Valve (5), which controls the speed of pressure building up and decaying in the timing circuit. From there the low flow rate passes through two connected ports in the 9 Second Delay Switch (8), to the piston head chamber of the Timing/Pressure Switch (6). Once the pressure inside this chamber climbs to approximately 21 PSI, the middle port of the Timing/Pressure Switch (6) (which is connected to the piston in Main Switch (2)) is connected to the low port (connected to air pressure power source P+ from Internal Regulator (1)), pressurizing the piston of the Main Switch (2) down and shutting off the gas flow.

The ventilator is now in the Expiratory phase. The gas inside the Timing/Pressure Switch (6) chamber then decays back through the Automatic Frequency Needle Valve (5), to the exhaust port located at the bottom of the Main Switch (2) out to ambient "A". Once the pressure inside the Timing/Pressure Switch (6) chamber decreases to a selected value, the middle port of the Timing/Pressure Switch (6) is switched to the upper port (connected to ambient), exhausting the gas in the piston head of the Main Switch (2) to ambient causing the Main Switch (2) to open again delivering flow to the patient. This cycle then repeats itself once more. The I:E ratio of 1:2 is controlled by the adjustable tension of the biasing spring on the Timing/Pressure Switch (6).

The Emergency Air Intake/Anti-Lockup Valve (7) has two functions. As an Anti-Lockup Valve, it is connected to the patient circuit through the Output Adaptor. When the circuit is in the Inspiratory or "ON" state, the Emergency Air Intake/ Anti-Lockup Valve (7) piston is pressurized against a biasing spring and the valve is closed delivering ventilation pressure to the patient. When the circuit is in the expiratory or "OFF" state, the Emergency Air Intake/ Anti-Lockup Valve (7) is de-pressurized and the biasing spring inside the valve opens it, exhausting any residual pressure in the patient breathing circuit (upstream to its diaphragm) to ambient ports on its outer diameter, thereby venting and eliminating what is known as "patient valve lockup" which opposes a patients effort during exhalation. The Emergency Air Intake function is covered in the following section.

Under conditions of power failure, the patient must be able to breathe spontaneously with airflow resistance at the patient connection port to inspiratory and expiratory flows not to exceed 6 cm-H2O @ 30 LPM.

The Emergency Air Intake/Anti-Lockup Valve (7) is connected to the patient circuit through the ventilator Output Adaptor. When the circuit is in the inspiratory or "ON" state, the Emergency Air Intake/ Anti-Lockup Valve (7) piston is pressurized against a biasing spring and the valve is closed. When the circuit is in the expiratory or "OFF" state, or if power failure occurs, the Emergency Air Intake/ Anti-Lockup Valve (7) is de-pressurized and the biasing spring inside the Valve opens it, allowing entrainment of air for the patient to breathe.

### CPR Compression Phase

The CPR Compression Phase provides 15 visual and/or audible signals over a 9 second time period for guiding the operator to pace chest compressions following the two Automatic cycles outlined above.

The CPR Compression Phase circuit consists of CPR-CP Check Valve (11), CPR-CP Cycles to Delay Needle Valve (12), CPR-CP Pressure Switch 1 (13), CPR-CP Pressure Switch 2 (14), CPR-CP Pressure Switch 3 (15), CPR-CP Output Switch (16), CPR-CP 15 Cycles Needle Valve (17), CPR-CP Sound Reed (18), CPR-CP Rotowink^{TM} Visual Indicator (19), 9 Second Delay Switch (8), CPR-CP Decay Needle Valve (20), CPR-CP Volume Needle Valve (21), and CPR-CP 9 Second Delay Needle Valve (22).

During each Automatic Circuit Inspiratory or "ON" phase, the Main Switch (2) output pressure is also directed through the CPR-CP Check Valve (11) and then to the CPR-CP Cycles to Delay Needle Valve (12). Each cycle pressure slowly builds up in the CPR-CP Pressure Switch 1 (13) (spring biased normally closed ports), depending on the setting of the CPR-CP Cycles to Delay Needle Valve (12). The CPR-CP Check Valve (11) prevents this pressure from decaying during the Automatic Circuit expiratory or "OFF" phase. Each cycle this pressure is stepped up, until the pressure has risen to approximately 21 PSI controlled by CPR-CP Cycles to Delay Needle Valve (12) which determines the number of automatic cycles that must occur to reach this switching pressure, the center port on Main Switch 2 then connects to the upper port which is connected to the pressure supply. This connects the pressure source to CPR-CP Pressure Switch 2 (14). The purpose of CPR-CP Pressure Switch 2 (14) is to permit injection of the supply pressure into the piston of the 9 Second Delay Switch (8) only when the Automatic circuit is in the Expiratory or "OFF" phase, otherwise this transfer of pressure would force the automatic circuit Inspiratory phase uncontrollably into an "OFF" state whenever CPR-CP Pressure Switch 1 (13) is activated. This component provides positive control of the initiation of the required 9 second delay after two automatic cycles. This pressure is transferred to the piston of the 9 Second Delay Switch (8) which causes an interruption of the automatic timing circuit via the switching ports located on it, to the piston of the Automatic Timing/Pressure Switch (6) and also to the CPR-CP Pressure Switch 2 (14). Before CPR-CP Pressure Switch 3 (15) is pressurized, its switching element on the left is closed and allows pressure to build up in CPR-CP Pressure Switch 1(13), and the ports on the right are connected to the pressure supply, pressurizing the CPR-CP Output Switch (16) piston against a biasing spring and closing it, preventing the compression pacing signals from activating. When CPR-CP Pressure Switch 3 (15) is pressurized, its right switching element connects the CPR-CP Output Switch (16) to CPR-CP 15 Cycles Needle Valve (17). The pressure in the CPR-CP Output Switch (16) then decays through the CPR-CP 15 Cycles Needle Valve (17), through the ports on CPR-CP Pressure Switch 3 (15) to CPR-CP Volume Needle Valve (21) and out to ambient through the CPR-CP Sound Reed (18). When pressure has decayed sufficiently in the CPR-CP Output Switch (16), the piston in it lifts and opens the valve supplying a flow of gas to the CPR-CP Volume Needle Valve (21), which reduces the flow rate needed to power the CPR-CP Sound Reed (18) generating an audible tone. The CPR-CP Volume Needle Valve (21) also generates a back pressure upstream that activates the CPR-CP Rotowink^{TM} Visual Indicator (19), and is also directed through the CPR-CP 15 Cycles Needle Valve (17) to the CPR-CP Output Switch (16) which pressurizes and shuts off when the pressure has built up sufficiently. This de-activates the CPR-CP Sound Reed (18) and the CPR-CP Rotowink ^{TM} Visual Indicator (19). The cycle then repeats itself in an oscillating fashion providing a cyclic audible and visual CPR compression pacer signal for approximately 15 cycles determined by the setting of CPR-CP 15 Cycles Needle Valve (17). When the pressure in the 9 Second Delay Switch (8) decays to ambient through CPR-CP 9 Second Delay Needle Valve (22) to ambient, the Automatic Circuit is restored and automatic cycling resumes for another two cycles. During the time period that CPR-CP Pressure Switch 3 (15) is pressurized, its left switching element connects and exhausts the pressure that built up in CPR-CP Pressure Switch 1 (13) during the two automatic cycles, to ambient through CPR-CP Decay Needle Valve (20).

### Face Mask Leakage Alarm

The purpose of this alarm circuit is to warn the operator that insufficient airway pressures are being generated during ventilations, due to either a poor face/mask seal, loose or damaged patient circuit parts.

The BSI (Breathing System Integrity) Alarm circuit consists of BSI Alarm Pressure Sensor Switch (31), BSI Alarm Pressure Switch (32), BSI Alarm Output Switch (33), BSI/MDP Alarm Sound Reed (34), BSI Alarm Time Delay Needle Valve (35), BSI Alarm Rotowink^{TM} Visual Indicator (36), BSI Alarm Frequency Needle Valve (37), BSI Alarm Volume Needle Valve (38), BSI Exhaust Check Valve (39), BSI Alarm Shut-Off Needle Valve (40) and Output Adaptor (41).

During the Automatic Circuit Inspiratory or "ON" phase, output pressure from the Main Switch (2) is applied to the downstream side of BSI Exhaust Check Valve (39) which substantially closes it but passes a controlled low flow rate of gas supplied via BSI Alarm Time Delay Needle Valve (35) to build up in the BSI Alarm Pressure Switch (32) and BSI Alarm Pressure Sensor Switch (31) (upstream side of BSI Exhaust Check Valve (39)). When no pressure exists in the BSI Alarm Pressure Switch, a biasing spring forces it's piston to the left position connecting the two centre ports, supplying another controlled low flow rate of gas via BSI Alarm Shut-Off Needle Valve (40) through the BSI Alarm Pressure Switch (32) into the top of the piston head in BSI Alarm Output Switch (33) causing it to shut off the flow of gas to the BSI/MDP Alarm Sound Reed (34). The alarm is in the off phase.

The BSI Alarm Pressure Sensor Switch (31) has a sensing conduit connected to the patient airway circuit via the Output Adaptor (41). When the Integrity of the Breathing System is present, including a good face mask seal, normal lung ventilation creates positive airway pressures in excess of 8 cmH2O during each cycle. The BSI Alarm Pressure Sensor Switch (31) triggers when the airway pressure reaches or exceeds 8 cmH2O and it exhausts the BSI circuit pressure that is slowly building up in the BSI Alarm circuit to ambient, keeping the BSI circuit pressure low thereby preventing the alarm from activating. If the Breathing System becomes disconnected from the patient or face/mask seal integrity is lost causing insufficient airway pressures to be generated during lung ventilation, the BSI Alarm Pressure Sensor Switch (31) will not sense enough airway pressure to trigger it, thereby allowing pressure to build up in the BSI Alarm circuit. As the pressure builds up in BSI Alarm Pressure Switch (32) to approximately 32 PSI (controlled by BSI Alarm Time Delay Needle Valve (35) to provide delay of approximately 1 second), the piston moves to the right and the two ports on the right are connected. The pressure in the piston head of the BSI Alarm Output Switch (33) decays through the BSI Alarm Pressure Switch (32) to the BSI Alarm Frequency Needle Valve (37) to the BSI Alarm Volume Needle Valve (38) and out to ambient through the BSI/MDP Alarm Sound Reed (34). When pressure has decayed sufficiently in the BSI Alarm Output Switch (33), the piston in it lifts and opens the valve supplying a flow of gas to the BSI Alarm Volume Needle Valve (38) which reduces the flow rate needed to power the BSI/MDP Alarm Sound Reed (34) generating an audible tone. The BSI Alarm Volume Needle Valve (38) also generates a back pressure upstream that activates the BSI Alarm Rotowink^{TM} Visual Indicator (36), and is also directed through the BSI Alarm Frequency Needle Valve (37) to the BSI Alarm Pressure Switch (32) and to the BSI Alarm Output Switch (33) which pressurizes and shuts off when the pressure has built up sufficiently. This de-activates the BSI/MDP Alarm Sound Reed (34) and the BSI Alarm Rotowink^{TM} Visual Indicator (36). The cycle then repeats itself in an oscillating fashion providing a cyclic audible and visual warning, until the deficiency in the Breathing System is corrected. Once the BSI Alarm is de-activated, a short delay of approximately 1 second will occur before the alarm is activated again.

During the Automatic Circuit Expiratory or "OFF" phase, output pressure from the Main Switch (2) is terminated and residual pressure at the downstream side of BSI Exhaust Check Valve (39) is exhausted to ambient. Any pressure built up in the BSI Alarm Circuit is also exhausted via the BSI Exhaust Check Valve (39) to the ambient port located at the bottom of the Main Switch (2). This prevents the BSI Alarm from activating during the Expiratory or "OFF" phase as well as during the CPR Compression phase.

### Airway Obstruction Alarm

The purpose of this circuit is to ensure that patient airway pressure does not exceed a preset value should the condition arise, by safely venting excess pressure to ambient and warning the operator with both audible and visual signals.

The Maximum Delivery Pressure (MDP) control circuit consists of a MDP Relief Valve (51), MDP Airway Pressure Sensor Switch (52), MDP Alarm Output Switch (53), MDP Alarm Rotowink^{TM} Visual Indicator (54), BSI/MDP Alarm Sound Reed (34), MDP Alarm Volume Needle Valve (55), and MDP Alarm Shut-Off Needle Valve (56).

This circuit is supplied by a small bleed of gas from the pressure source via MDP Alarm Shut-Off Needle Valve (56), which is fed into the MDP Alarm Output Switch (53) and to the MDP Airway Pressure Sensor Switch (52). The MDP Airway Pressure Sensor Switch (52) consists of a sensing diaphragm and a tilt lever switch. The tilt lever switch is spring biased in the normally closed position, and it contains the pressure that is slowly building up in this circuit. The MDP Alarm Output Switch (53) is a high flow pressure switch spring biased in the normally open position and is supplied by the Internal Regulator (1). When pressure rises in this circuit to a sufficient level, the MDP Alarm Output Switch (53) piston is pressurized and output flow from it is terminated, preventing activation of the alarm signals.

The patient circuit is in communication with a MDP Relief Valve (51). It consists of a biasing spring that applies downward force onto a plate backed silicone diaphragm that in turn rests on a circular seat that is in communication with the patient circuit. The maximum delivery pressure is adjusted by varying the tension applied to the spring.

When the airway pressure applied to the underside (or patient airway circuit side) of the diaphragm plate is greater than a preset value, the diaphragm will move upwards against the biasing spring and vent the excess airway pressure to ambient through multiple exhaust ports located on the outside of the seat in the body. A small portion of the vented gas from the MDP Relief Valve (51) is diverted by a separate internal jet (which is normally closed against a central rubber seat on the diaphragm assembly, until the Pressure Relief Valve (51) is activated) to the MDP Airway Pressure Sensor Switch (52). Pressure applied to the top of the diaphragm inside of it generates a downward force that opens the spring biased tilt lever, venting or exhausting the circuit pressure that is being contained by it, through an ambient port located on the underside of the diaphragm, on the side of the body. When pressure has decayed sufficiently in the MDP Alarm Output Switch (53), the piston in it lifts and opens the valve supplying a flow of gas to the MDP Alarm Volume Needle Valve (55) which reduces the flow rate needed to power the BSI/MDP Alarm Sound Reed (34) generating an audible warning tone. The MDP Alarm Volume Needle Valve (55) also generates a back pressure upstream that activates the MDP Alarm Rotowink^{TM} Visual Indicator (54). When the airway pressure has returned to a safe level, the tilt lever in the MDP Airway Pressure Sensor Switch (52) closes and once again contains the pressure slowly building up in the circuit. When the pressure has built up sufficiently, the MDP Alarm Output Switch (53) pressurizes and shuts off the flow of gas. This de-activates the BSI/MDP Alarm Sound Reed (34) and the MDP Alarm Rotowink^{TM} Visual Indicator (54).

Although the above description and accompanying drawings relate to a specific preferred embodiment as presently contemplated by the inventor, it will be understood that the invention in its broad aspect includes mechanical and functional equivalents of the elements described and illustrated.

## Claims

1. An automatic ventilator for cardio-pulmonary resuscitation (CPR) comprising:
an automatic ventilating circuit adapted for delivering two cycles of positive pressure breathable gas flow ventilation to a patient's airway; and
a CPR timing circuit adapted to emit timed signals over a CPR period, after said two cycles, to guide an operator to time chest compressions applied to a patient.

2. The automatic ventilator according to claim 1, comprising:
a breathing system integrity alarm circuit including a BSI alarm signal emitted when the gas pressure in the airway during inspiration is below a predetermined minimum pressure.

3. The automatic ventilator according to claim 1, comprising:
a maximum delivery pressure alarm circuit including a MDP alarm signal emitted when the gas pressure in the airway during inspiration is above a predetermined maximum delivery pressure.

4. The automatic ventilator according to any one of claims 2 and 3, wherein the CPR timing circuit emits timed signals including at least one of: a verbal signal; an audible signal; and a visual signal.

5. The automatic ventilator according to claim 1, wherein at least one of the BSI alarm circuit and the MDP alarm circuit include an alarm selected from the group consisting of: an audible alarm; and a visual alarm.

6. The automatic ventilator according to claim 1, wherein said cycles of positive pressure breathable gas flow have an inspiration time of about 2 seconds.

7. The automatic ventilator according to claim 1, wherein said cycles of positive pressure breathable gas flow have an expiration time of about 4 seconds.

8. The automatic ventilator according to claim 1, wherein said cycles deliver a tidal volume of about 0.5L per cycle.

9. The automatic ventilator according to claim 1, wherein said CPR period has a time of about 9 seconds.

10. The automatic ventilator according to claim 9, wherein fifteen signals are emitted during the CPR period.
